# EUROPÄISCHE PATENTSCHRIFT

(11) **EP 1 519 696 B1**
(45) Veröffentlichungstag und Bekanntmachung des Hinweises auf die Patenterteilung: **27.07.2016**
(21) Anmeldenummer: 03740064.5
(22) Anmeldetag: 04.06.2003
(51) Int. Cl.: A61F 2/50, A61F 2/80

(54) **LINER FÜR BEIN- ODER ARMSTÜMPFE**
LINER
ELEMENT DE GARNISSAGE

(30) Priorität: 04.06.2002 DE 20208592 U
(43) Veröffentlichungstag der Anmeldung: 06.04.2005
(73) Patentinhaber: medi prosthetics GmbH, 95448 Bayreuth (DE)
(72) Erfinder: KURTH, Christoph, 95448 Bayreuth (DE)
(74) Vertreter: Blaumeier, Jörg
(86) Internationale Anmeldenummer: PCT/DE2003/001835
(87) Internationale Veröffentlichungsnummer: WO 2003/101351

(56) Entgegenhaltungen:
- WO-A-98/49977
- DE-A- 2 329 929
- DE-A- 10 012 929
- DE-U1- 8 003 452
- US-A- 4 653 473
- US-A- 4 664 118
- US-A- 5 443 525
- US-A- 5 534 034
- US-A- 6 014 585

## Beschreibung

Die vorliegende Erfindung betrifft eine Stützhülse für Bein- oder Armstümpfe, nämlich einen Liner, mit den Merkmalen des Oberbegriffs des Anspruchs 1.

Liner für Prothesen wie Beinprothesen, Armprothesen oder dergleichen, bestehend aus einer den Stumpf von dessen distalem Ende aus umschließenden Hülse aus einem elastischen, elektrisch isolierenden Material wie Silikon, Polyurethan oder dergleichen sind bekannt (WO 88/00032, WO 98/49977, EP 0 976 371 A1). Solche Liner werden als gepolsterte Verbindung zwischen Stumpf und Prothesenschaft eingesetzt, werden aber auch postoperativ verwendet, um in der Abheilphase nach Amputationen abschwellend und formgebend zu wirken. Es ist weiter bekannt, dass nach Amputationen so genannter Phantomschmerz auftreten kann, der mit einer elektromagnetischen Abschirmung des Stumpfes, z.B. durch ein elektrisch leitendes Textilmaterial positiv beeinflusst werden kann (US 4,653,473 A).

Aufgabe der vorliegenden Erfindung ist es, einen Liner zu schaffen, der einen solchen lindernden Effekt gegenüber Stumpfschmerzen besitzt, jedoch ohne die eigentlichen Qualitäten eines Liners einzubüßen.

Diese Aufgabe wird mit den Merkmalen des kennzeichnenden Teils des Anspruchs 1 gelöst. Fortbildungen und vorteilhafte Ausführungen der Erfindung sind in den weiteren Ansprüchen umfaßt.

Erfindungsgemäß ist eine Stützhülse für Bein- oder Armstümpfe, nämlich Liner, bestehend aus einer den Stumpf von dessen distalem Ende aus umschließenden Hülse aus einem elastischen, elektrisch isolierenden Material, dadurch gekennzeichnet, dass die Hülse wenigstens teilweise eine umlaufende Schicht aus einem leitfähigen Material aufweist.

Nach einer ersten Ausführung der Erfindung ist die elektrisch isolierende Schicht außen auf der Hülse aufgebracht und besteht aus einer Schicht aus einem elastischen, leitfähig ausgerüsteten Textilmaterial oder Kunststoff. Beispielsweise kann es sich um Material handeln, das mit einem Metall bedampft wurde. Wiederum beispielsweise kann der Kunststoff als Kleber aufgebracht sein:

Nach einer zweiten Ausführung der Erfindung ist die Schicht eine von dem elastischen Material eingeschlossene, am distalen Ende des Liners beginnende textile mechanische Verstärkung (Matrix) aus einem elektrisch leitfähigen Material, wobei die Matrix beispielsweise aus geordneten oder ungeordneten Fäden eines leitfähigen Materials besteht.

Nach einer besonders vorteilhaften Ausführung der Erfindung ist zwischen dem Stumpf und der Schicht aus einem leitfähigen Material ein leitfähiger Bereich angeordnet, wobei der leitfähige Bereich vorzugsweise am distalen Ende des Stumpfes angeordnet ist.

Nach einer Variante dieser bevorzugten Ausführung der Erfindung weist der Liner an seinem distalen Ende eine Linertasse und ggf. einen Pinadapter zur Verbindung mit einer Prothese auf, wobei die Linertasse und/oder der Pinadapter aus einem elektrisch leitfähigen Material bestehen, oder mit einem solchen ausgerüstet sind. Die Linertasse und/oder der Pinadapter stehen mit der Schicht aus elektrisch leitfähigen Material in Kontakt, wobei der leitfähige Bereich zwischen dem Stumpf und der Linertasse und/oder dem Pinadapter angeordnet ist. Wird der Liner ausschließlich zur postoperativen Behandlung eingesetzt, können Linertasse und Pinadapter entfallen.

Vorzugsweise ist zwischen dem Stumpf und dem leitfähigen Bereich ein elektrischer Widerstand von < 10⁵ Ohm vorhanden.

Im Folgenden wird die Erfindung anhand einer Zeichnung beispielhaft näher beschrieben.

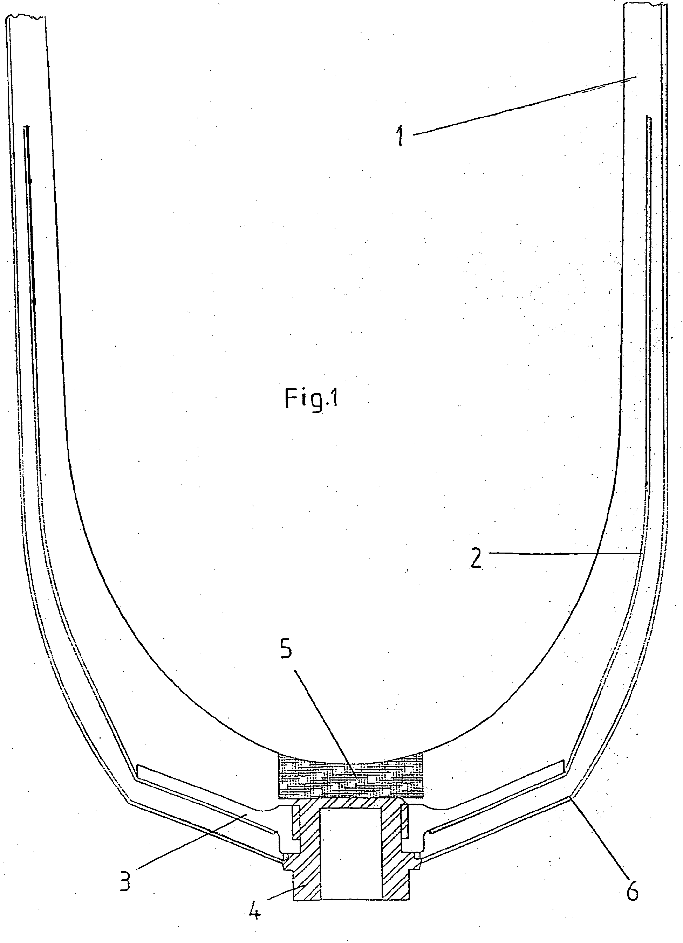

Fig. 1 zeigt einen Liner 1 aus einem elektrisch isolierenden Silikonmaterial zur Aufnahme eines Beinstumpfs. Der Liner 1 ist mit einem Überzug 6 aus einem textilen Material versehen. In den Liner 1 ist eine Schicht 2 eines elektrisch leitenden Materials, beispielsweise aus Metallfäden eingebettet. Andern distalen Ende des Liners befindet sich ein an sich bekannter Pinadapter 4 für die Verbindung mit einer Unterschenkelprothese. Über eine sogenannte Linertasse 3 wird der Liner 1 von unten gestützt. Ein derartiger Aufbau ist z.B. aus der DE 100 12 929 A1 bekannt. Der Pinadapter 4 und die Linertasse 3 bestehen aus Metall und sind elektrisch leitfähig. Die Linertasse 3 steht mit der eingearbeiteten Matrix 2 aus elektrisch leitendem Material in Kontakt. Zur Herstellung einer elektrisch leitenden Verbindung mit dem distalen Ende des Stumpfs ist über dem Pinadapter 4 ein elektrisch leitfähiger Bereich 5 angeordnet, der beispielsweise aus einem Filz aus einem elastomeren Material besteht, das leitfähig ausgerüstet ist (metallisch bedampft, Leitruß enthaltend) oder von leitfähigen Fäden aus Graphit, Metall oder dergleichen durchzogen ist, wobei der elektrisch leitende Bereich 5 und der Liners im wesentlichen die gleiche Materialhärte aufweisen.

## Patentansprüche

1. Liner für Bein- oder Armstümpfe, bestehend aus einer den Stumpf von dessen distalem Ende aus umschließenden Hülse aus einem elastischen, elektrisch isolierenden Material, **dadurch gekennzeichnet, dass** die Hülse (1) wenigstens teilweise eine umlaufende Schicht (2) aus einem leitfähigen Material aufweist, die außen auf der Hülse aufgebracht ist oder eine von dem elastischen Material eingeschlossene Matrix aus einem leitfähigen Material ist.

2. Liner nach Anspruch 1, **dadurch gekennzeichnet, dass** die außen auf die Hülse aufgebrachte Schicht aus einem elastischen, leitfähig ausgerüsteten Textilmaterial oder Kunststoff besteht.

3. Liner nach Anspruch 1, **dadurch gekennzeichnet, dass** die Matrix aus geordneten oder ungeordneten Fäden eines leitfähigen Materials besteht.

4. Liner nach einem der vorhergehenden Ansprüche, **dadurch gekennzeichnet, dass** zwischen dem Stumpf und der Schicht aus einem leitfähigen Material ein leitfähiger Bereich (5) angeordnet ist.

5. Liner nach Anspruch 4, **dadurch gekennzeichnet, dass** der leitfähige Bereich am distalen Ende des Stumpfes angeordnet ist.

6. Liner nach einem der vorhergehenden Ansprüche, **dadurch gekennzeichnet, dass** der Liner an seinem distalen Ende eine Linertasse (3) und einen Pinadapter (4) zur Verbindung mit einer Prothese aufweist.

7. Liner nach Anspruch 6, **dadurch gekennzeichnet, dass** die Linertasse und/oder der Pinadapter aus einem elektrisch leitfähigen Material bestehen oder mit einem solchen ausgerüstet sind.

8. Liner nach Anspruch 7, **dadurch gekennzeichnet, dass** die Linertasse und/oder der Pinadapter mit der Schicht aus elektrisch leitfähigen Material in Kontakt stehen.

9. Liner nach Anspruch 5 und 8, **dadurch gekennzeichnet, dass** der leitfähige Bereich zwischen dem Stumpf und der Linertasse und/oder dem Pinadapter angeordnet ist.

10. Liner nach einem der Ansprüche 4 - 9, **dadurch gekennzeichnet, dass** zwischen dem Stumpf und dem leitfähigen Bereich ein elektrischer Widerstand von < 10⁵ Ohm vorhanden ist.

11. Liner nach einem der Ansprüche 6 - 10, **dadurch gekennzeichnet, dass** zwischen der Linertasse und dem Prothesenschaft Verriegelungsmittel vorhanden sind, wobei die Verriegelungsmittel elektrisch leitend oder elektrisch isolierend ausgeführt sind.

## Claims

1. Liner for leg or arm stumps, consisting of a sleeve which encloses the stump, starting from the distal end thereof, and which is made of an elastic, electrically insulating material, **characterized in that** the sleeve (1) has at least in parts a surrounding layer (2) made of a conductive material, which layer is applied to the outside of the sleeve or is a matrix of conductive material enclosed by the elastic material.

2. Liner according to Claim 1, **characterized in that** the layer applied to the outside of the sleeve is made of an elastic, conductive textile material or plastic.

3. Liner according to Claim 1, **characterized in that** the matrix consists of ordered or unordered filaments of a conductive material.

4. Liner according to one of the preceding claims, **characterized in that** a conductive area (5) is arranged between the stump and the layer made of a conductive material.

5. Liner according to Claim 4, **characterized in that** the conductive area is arranged at the distal end of the stump.

6. Liner according to one of the preceding claims, **characterized in that** the liner has, at its distal end, a liner cup (3) and a pin adapter (4) for connection to a prosthesis.

7. Liner according to Claim 6, **characterized in that** the liner cup and/or the pin adapter are made of an electrically conductive material or are provided with such.

8. Liner according to Claim 7, **characterized in that** the liner cup and/or the pin adapter are in contact with the layer made of electrically conductive material.

9. Liner according to Claims 5 and 8, **characterized in that** the conductive area is arranged between the stump and the liner cup and/or the pin adapter.

10. Liner according to one of Claims 4 to 9, **characterized in that** an electrical resistance of < 10⁵ Ohm is present between the stump and the conductive area.

11. Liner according to one of Claims 6 to 10, **characterized in that** locking means are present between the liner cup and the prosthesis socket, wherein the locking means are electrically conductive or electrically insulating.

## Revendications

1. Elément de garnissage pour moignons de jambe ou de bras, se composant d'une manchette en un matériau élastique, électriquement isolant, qui entoure le moignon à partir de son extrémité distale, **caractérisé en ce que** la manchette (1) présente au moins en partie une couche enveloppante (2) en un matériau conducteur, qui est appliquée extérieurement sur la manchette ou qui est une matrice en un matériau conducteur enrobée par le matériau élastique.

2. Elément de garnissage selon la revendication 1, **caractérisé en ce que** la couche appliquée extérieurement sur la manchette se compose d'une matière textile élastique ou d'une matière plastique rendue conductrice.

3. Elément de garnissage selon la revendication 1, **caractérisé en ce que** la matrice de compose de fils ordonnés ou désordonnés d'un matériau conducteur.

4. Elément de garnissage selon l'une quelconque des revendications précédentes, **caractérisé en ce qu'**une zone conductrice (5) est agencée entre le moignon et la couche en un matériau conducteur.

5. Elément de garnissage selon la revendication 4, **caractérisé en ce que** la zone conductrice est agencée à l'extrémité distale du moignon.

6. Elément de garnissage selon l'une quelconque des revendications précédentes, **caractérisé en ce que** l'élément de garnissage présente à son extrémité distale une cuvette d'élément de garnissage (3) et un adaptateur de broche (4) pour l'assemblage à une prothèse.

7. Elément de garnissage selon la revendication 6, **caractérisé en ce que** la cuvette d'élément de garnissage et/ou l'adaptateur de broche sont constitués d'un matériau électriquement conducteur ou en sont équipés.

8. Elément de garnissage selon la revendication 7, **caractérisé en ce que** la cuvette d'élément de garnissage et/ou l'adaptateur de broche sont en contact avec la couche en matériau électriquement conducteur.

9. Elément de garnissage selon la revendication 5 et 8, **caractérisé en ce que** la zone conductrice est agencée entre le moignon et la cuvette d'élément de garnissage et/ou l'adaptateur de broche.

10. Elément de garnissage selon l'une quelconque des revendications 4 à 9, **caractérisé en ce qu'**il se trouve une résistance électrique de < 10⁵ ohms entre le moignon et la zone conductrice.

11. Elément de garnissage selon l'une quelconque des revendications 6 à 10, **caractérisé en ce qu'**il se trouve des moyens de verrouillage entre la cuvette d'élément de garnissage et la tige de prothèse, dans lequel les moyens de verrouillage sont électriquement conducteurs ou électriquement isolants.
